(19) Europäisches Patentamt
European Patent Office
Office européen des brevets

(11) Veröffentlichungsnummer: **0 003 558**
**B1**

(12) # EUROPÄISCHE PATENTSCHRIFT

(45) Veröffentlichungstag der Patentschrift: **21.10.81**

(21) Anmeldenummer: **79100284.3**

(22) Anmeldetag: **31.01.79**

(51) Int. Cl.³: **C 07 D 473/34,**
A 61 K 31/52 //C07D211/58

(54) **9-(3-Piperidinopropyl)-purin-Derivate, Verfahren zu deren Herstellung, deren Verwendung sowie diese Verbindungen enthaltende Arzneimittel.**

(30) Priorität: **02.02.78 DE 2804416**

(43) Veröffentlichungstag der Anmeldung:
**22.08.79 Patentblatt 79/17**

(45) Bekanntmachung des Hinweises auf die Patenterteilung:
**21.10.81 Patentblatt 81/42**

(84) Benannte Vertragsstaaten:
**BE CH DE FR GB IT LU NL SE**

(56) Entgegenhaltungen:
**DE - A - 2 550 000**

(73) Patentinhaber: **BOEHRINGER MANNHEIM GMBH**
**Sandhofer Strasse 112-132 Postfach 31 01 20**
**D-6800 Mannheim 31-Waldhof (DE)**

(72) Erfinder: **Friebe, Walter-Gunar, Dr.**
**Oberstrasse 13**
**D-6100 Darmstadt (DE)**
Erfinder: **Thiel, Max, Dr.**
**S 6, 35**
**D-6800 Mannheim 1 (DE)**
Erfinder: **Roesch, Androniki, Dr.**
**Am Oberen Luisenpark 22**
**D-6800 Mannheim 1 (DE)**
Erfinder: **Wilhelms, Otto-Henning, Dr.**
**Odenwaldstrasse 25/2**
**D-6941 Weinheim-Rittenweier (DE)**
Erfinder: **Schaumann, Wolfgang Prof., Dr.**
**Mönchhofstrasse 58**
**D-6900 Heidelberg (DE)**

**EP 0 003 558 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# 9-(3-Piperidinopropyl)-purin-Derivate, Verfahren zu deren Herstellung, deren Verwendung sowie diese Verbindungen enthaltende Arzneimittel

Die vorliegende Erfindung betrifft neue 9 - (3 - piperidinopropyl) - purin - Derivate, Verfahren zu deren Herstellung sowie diese Verbindungen enthaltende Arzneimittel.

Die neuen Verbindungen sowie ihre pharmakologisch unbedenklichen Salze unterdrücken die Freisetzung und Wirkung von Histamin und können damit antiallergisch, entzündungswidrig und antiödematös wirken.

In der DE—A—25 50 000 sind 9 - (3 - Piperidinopropyl) - purin - Derivate mit antiallergischer, entzündungswidriger, antiödematöser und antihypertensiver Wirkung beschrieben, deren Piperidin-Gruppe in 4-Stellung durch einen Phenoxymethyl-Rest substituiert ist.

Die vorliegende Erfindung betrifft Piperidinoalkyl-Derivate des Purins der allgemeinen Formel I

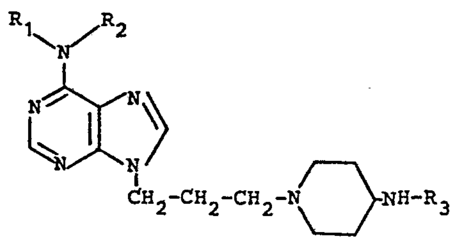

(I)

in welcher

$R_1$ Wasserstoff oder eine Alkyl-Gruppe, mit 1 bis 6 Kohlenstoffatomen, $R_2$ Wasserstoff, eine Aryl- oder eine Alkyl-Gruppe mit 1 bis 6 Kohlenstoffatomen die gegebenenfalls durch Hydroxyl substituiert sein kann, wobei $R_1$ und $R_2$ auch zusammen mit dem Stickstoffatom einen heterocyclischen Ring bilden können, und

$R_3$ eine Acyl-Gruppe oder Wasserstoff

bedeuten, sowie deren Salze mit pharmakologisch verträglichen Säuren.

Weiterhin betrifft die Erfindung pharmazeutische Präparate mit einem Gehalt an Verbindungen der allgemeinen Formel I sowie die Verwendung von Verbindungen der allgemeinen Formel I zur Herstellung solcher Präparate.

Die Alkyl-Gruppen der Substituenten $R_1$ und $R_2$ können geradkettig oder verzweigt sein und bevorzugt 1—4 Kohlenstoffatome enthalten. Heterocyclische Ringe, die $R_1$ und $R_2$ zusammen mit dem Stickstoffatom bilden können, sind beispielsweise der Tetramethylenimino-, der Pentamethylenimino- und Hexamethylenimino- Ring.

Die Acyl-Gruppen des Substituenten $R_3$ können Niederalkanoyl-Gruppen, die gegebenenfalls ein- oder mehrfach durch Halogen oder durch Aryl substituiert sind, durch Aryl substituierte Niederalkenoyl-Gruppen, vorzugsweise die Cinnamoyl oder 3,4-Dimethoxy-cinnamoyl-Gruppe oder carbocyclische oder heterocyclische Aroyl-Gruppen bedeuten, die gegebenenfalls durch Halogen, Hydroxyl, Niederalkyl, Niederalkoxy, Niederalkoxycarbonyl, Niederacyloxy, Carboxyl, Nitro, Amino, Nitril, Trifluormethyl, Carbamoyl oder Benzyl substituiert sein können, bedeuten.

Heterocyclische Aroyl-Gruppen sind beispielsweise der Furancarbonyl-, der Thiophencarbonyl- und der Pyridincarbonyl- Rest, carbocyclische Aroyl-Gruppen beispielsweise der Benzoyl-Rest.

Des weiteren kann $R_3$ des Säurerest einer Cycloalkancarbonsäure darstellen, wobei unter Cycloalkyl vorzugsweise der Cyclopropyl-, Cyclobutyl-, Cyclopentyl-, Cyclohexyl- und Cycloheptyl-Rest verstanden wird.

Ferner sind unter $R_3 = $Acyl auch Säurereste von Sulfonsäuren wie z.B. Benzolsulfonsäure und Methansulfonsäure zu verstehen. Der Ausdruck Aryl in der Definition der Substituenten $R_2$ und $R_3$ bedeutet in allen Fällen vorzugsweise Phenyl- oder Naphthyl.

Als Halogenatome kommen Fluor, Chlor und Brom in Frage.

Ausser den in den Beispielen genannten Verbindungen sind Gegenstand der vorliegenden Erfindung insbesondere alle Substanzen, die jede mögliche Kombination der in den Beispielen genannten Substituenten aufweisen.

Die neuen Verbindungen, die in 4-Stellung des Piperidin-Restes durch eine gegebenenfalls acylierte Aminogruppe substituiert sind, weisen im Vergleich zum nächstliegenden Stande der Technik bessere antiallergische, entzündungswidrige und antiödematöse Eigenschaften auf.

Zum Beweis wurden im pharmakologischen Test die passive cutane anaphylaktische Reaktion (PCA-Werte) für das Handelsprodukt Diethylcarbamazin (1 - Diethyl - carbamoyl - 4 - methyl - piperazin) und für die Verbindung des Beispiels 2 1) (6 - Dimethylamino - 9 - [3 - (4 - benzamido - piperidino) - propyl] - purin) ermittelt. Um eine vergleichbare Inhibierung der passiven cutanen anaphylaktischen Reaktion zu erreichen, muss von dem Handelsprodukt vierzigmal mehr als von der erfindungsgemässen Verbindung eingesetzt werden.

Anhand einer geeigneten Auswahl aus den beanspruchten Verbindungen konnte aufgezeigt

werden, dass auch die anderen erfindungsgemässen Verbindungen eine ebenso gute Inhibierung der passiven cutanen anaphylaktischen Reaktion bewirken.

Das erfindungsgemässe Verfahren zur Herstellung der Verbindungen der Formel I ist dadurch gekennzeichnet, dass man in an sich bekannter Weise eine Verbindung der allgemeinen Formel II

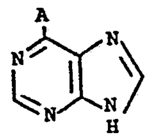

(II)

in der
A einen reaktiven Rest oder die

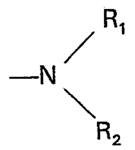

-Gruppe, in welcher $R_1$ und $R_2$ die obengenannte Bedeutung haben,
bedeutet,
mit einer Verbindung der allgemeinen Formel III

$$X—CH_2—CH_2—CH_2—Y \qquad (III),$$

in der
X und Y reaktive Reste bedeuten und einer Verbindung der allgemeinen Formel IV

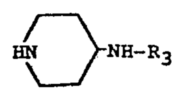

(IV)

in der $R_3$ die obengenannte Bedeutung hat,
umsetzt,
wobei für den Fall, dass A eine reaktive Gruppe bedeutet, die

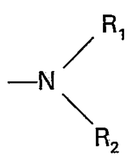

-Gruppe nachträglich eingeführt werden muss, anschliessend gewünschtenfalls die Gruppe $R_3$ durch Verseifung und folgende Acylierung mit einer Verbindung $R_3$—Z, wobei Z einen reaktiven Rest bedeutet, gegen eine andere, durch den Anspruch umfasste Gruppe $R_3$ austauscht,
gegebenenfalls eine Nitrogruppe durch Hydrierung in eine Aminogruppe überführt
und anschliessend das erhaltene Reaktionsprodukt gewünschtenfalls in ein pharmakologisch verträgliches Salz überführt.

Als reaktive Reste X und Y der Verbindungen der allgemeinen Formel III kommen Chlor, Brom, Mesyloxy und Tosyloxy in Frage. Unter einem reaktiven Rest A der allgemeinen Formel II versteht man Halogen, vorzugsweise Chlor und Brom, sowie die Alkylmercapto- oder Benzylmercapto-Gruppe. Reaktive Reste Z können alle Reste sein, die in der Peptid-Chemie zur Aktivierung von Carbonsäuren Verwendung finden, beispielsweise Halogenatome, die Azido-Gruppe, Alkyloxy-, Aryloxy- und Acyloxy-Gruppen.

Das erfindungsgemässe Verfahren führt man vorzugsweise so durch, dass man zunächst Verbindungen der allgemeinen Formel III mit Verbindungen der allgemeinen Formel IV kondensiert und das erhaltene Reaktionsproduckt isoliert. Dieses Zwischenprodukt wird dann mit einer Verbindung der allgemeinen Formel II zur Reaktion gebracht. Die Reaktion erfolgt zweckmässig in alkalischem Medium, vorzugsweise in einem niederen Alkohol wie z.B. in Isopropanol in Anwesenheit von Natriumisopropanolat. Unter den genannten Bedingungen erhält man die Verbindungen der Formel I neben geringen Mengen der isomeren, in 7-Stellung substituierten Derivate, die jedoch durch Umkristallisieren der Reaktionsprodukte entfernt werden können (über 9-Substitution des Adenins in alkalischem Medium

vgl. auch "The chemistry of heterocyclic compounds": Fused pyrimidines, Part II, Purines, Wiley-Interscience, Seite 342).

Eine andere Variante besteht darin, zunächst Verbindungen der allgemeinen Formel II mit Verbindungen der allgemeinen Formel III zur Reaktion zu bringen; anschliessend wird das erhaltene Reaktionsgemisch mit Verbindungen der allgemeinen Formel IV zum gewünschten Endprodukt der allgemeinen Formel I umgesetzt.

Für den Fall, dass A eine reaktive Gruppe ist, muss die

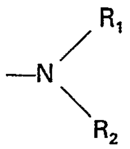

-Gruppe nachträglich eingeführt werden. Dies geschieht nach allgemein bekannten Verfahren aus dem Gebiet der Purin-Chemie (vgl. loc. cit. Seite 159).

Eine nachträgliche Umwandlung der Gruppe $R_3$ in der allgemeinen Formel I in eine andere Gruppe $R_3$ erfolgt bevorzugt als Austausch eines Acylrestes $R_3$ gegen einen anderen Rest $R_3$. Hierzu werden Verbindungen der Formel I zunächst in saurem Milieu verseift und die erhaltenen Zwischenprodukte vorzugsweise in Gegenwart eines säurebindenden Mittels nach bekannten Methoden acyliert. Die als Zwischenprodukte erhaltenen 9 - [3 - (4 - Amino - piperidino) - propyl] - adenin - Derivate sind ebenfalls neue Verbindungen.

Des weiteren kann in Verbindungen der Formel I, in denen der Acylrest $R_3$ eine Nitro-Gruppe enthält, die Nitro-Gruppe durch bekannte Methoden in eine Amino-Gruppe umgewandelt werden, z.B. katalytische Hydrierung.

Die Verbindungen der Formeln II, III und IV sind aus der Literatur bekannt oder können von bekannten Verbindungen ausgehend nach trivialen Methoden leicht hergestellt werden.

Die Verbindungen der allgemeinen Formel IV sind z.T. neue Verbindungen. Sie können nach an sich bekannten Verfahren hergestellt werden, indem man beispielsweise ein 4-Aminopiperidin, das am Ringstickstoff eine Schutzgruppe trägt, entsprechend acyliert und anschliessend die Schutzgruppe abspaltet.

Die pharmakologisch verträglichen Salze erhält man in üblicher Weise, z.B. durch Neutralisation der Verbindungen der Formel I mit nichttoxischen anorganischen oder organischen Säuren wie z.B. Salzsäure, Schwefelsäure, Phosphorsäure, Bromwasserstoffsäure, Essigsäure, Milchsäure, Zitronensäure, Aepfelsäure, Salicylsäure, Malonsäure, Maleinsäure oder Bernsteinsäure.

Die erfindungsgemässen neuen Substanzen der Formel I und ihre Salze können in flüssiger oder fester Form enteral und parenteral appliziert werden. Hierbei kommen alle üblichen Applikationsformen in Frage, beispielsweise Tabletten, Kapseln, Dragees, Sirupe, Lösungen, Suspensionen etc. Als Injektionsmedium kommt vorzugsweise Wasser zur Anwendung, welches die bei Injektionslösungen üblichen Zusätze wie Stabilisierungsmittel, Lösungsvermittler und Puffer enthält. Derartige Zusätze sind z.B. Tartrat- und Citrat-Puffer, Aethanol, Komplexbildner (wie Aethylendiamintetraessigsäure und deren nichttoxische Salze), hochmolekulare Polymere (wie flüssiges Polyaethylenoxid) zur Viskositätsregelung. Flüssige Trägerstoffe für Injektionslösungen müssen steril sein und werden vorzugsweise in Ampullen abgefüllt. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyaethylenglykole); für orale Applikation geeignete Zubereitungen können gewünschtenfalls Geschmacks- und Süsstoffe enthalten.

In den folgenden Beispielen wird die Herstellung der erfindungsgemässen Verbindungen sowie einer pharmazeutischen Zubereitungsform näher beschrieben.

Bevorzugt im Sinne der Anmeldung sind ferner dei folgenden Verbindungen:

9-(3-[4-(2-Chlor-benzamido)-piperidino]-propyl)-adenin
9-(3-[4-(3-Chlor-benzamido)-piperidino]-propyl)-adenin
9-(3-[4-(3-Methoxy-benzamido)-piperidino]-propyl)-adenin
9-(3-[4-(4-Methoxy-benzamido)-piperidino]-propyl)-adenin
9-(3-[4-(2-Acetoxy-benzamido)-piperidino]-propyl)-adenin
9-(3-[4-(3-Methyl-benzamido)-piperidino]-propyl)-adenin
9-(3-[4-(4-Benzyl-benzamido)-piperidino]-propyl)-adenin
9-(3-[4-(4-Cyano-benzamido)-piperidino]-propyl)-adenin
9-(3-[4-(4-Ethoxycarbonyl-benzamido)-piperidino]-propyl)-adenin
9-(3-[4-(4-Carboxy-benzamido)-piperidino]-propyl)-adenin
$N^6$-Ethyl-9-[3-(4-benzamido-piperidino)-propyl]-adenin
$N^6$-Phenyl-9-[3-(4-benzamido-piperidino)-propyl]-adenin
6-Pyrrolidino-9-[3-(4-benzamido-piperidino)-propyl]-purin

9-[3-(4-Trifluoroacetamido-piperidino)-propyl]-adenin
9-[3-(4-Butyramido-piperidino)-propyl]-adenin
9-[3-(4-Cyclopentancarbonylamido-piperidino)-propyl]-adenin

sowie deren Salzen mit pharmakologisch verträglichen Säuren.

Beispiel 1

*9-[3-(4-Benzamido-piperidino)-propyl]-adenin*

Zu der Lösung von 0.8 g (0.035 mol) Natrium in 125 ml Isopropanol gibt man 4.7 g (0.035 mol) Adenin, erhitzt 10 Minuten unter Rückfluss, kühlt ab und fügt 11.2 g (0.04 mol) 3 - (4 - Benzamido - piperidino) - propylchlorid in 50 ml Isopropanol zu. Nach 6 Stunden Rühren unter Rückfluss engt man im Vakuum ein, nimmt in Methylenchlorid auf, wächst mit 2 N Natronlauge und anschliessend mit Wasser, engt ein und kristallisiert aus Ethanol um.

Man erhält 7.1 g 9 - [3 - (4 - Benzamido - piperidino) - propyl] - adenin (54% d. Th.) vom Schmp. 213—214°C.

Das als Reaktionskomponente verwendete 3 - (4 - Benzamido - piperidino) - propylchlorid wird wie folgt hergestellt:

Eine Mischung von 20.4 g (0.1 mol) 4 - Benzamido - piperidin, 15.7 g (0.1 mol) 1 - Brom - 3 - chlor - propan, 30.3 g (0.3 mol) Triethylamin und 200 ml Tetrahydrofuran wird 6 Stundan zum Rückfluss erhitzt, filtriert, das Filtrat im Vakuum eingeengt, der Rückstand mit Essigester extrahiert und der Extrakt eingeengt.

Man erhält 13.0 g 3 - (4 - Benzamido - piperidino) - propylchlorid (46.5% d. Th.) vom Schmp. 128—130°C.

In entsprechender Weise werden die in den folgenden Beispielen verwendeten substituierten Acylamidopiperidinopropylchloride hergestellt.

Beispiel 2

In analoger Weise wie in Beispiel 1 beschrieben erhält man:

| | Bezeichnung | | Ausbeute % | Schmp. °C (Lösungsmittel) |
|---|---|---|---|---|
| a) | 9-(3-[4-(2-Fluor-benzamido)-piperidino]-propyl)-adenin aus Adenin und 3-[4-(2-Fluor-benzamido)-piperidino]-propylenchlorid | aus | 50 | 180—181 (Isopropanol) |
| b) | 9-(3-[4-(4-Fluor-benzamido)-piperidino]-propyl)-adenin aus Adenin und 3-[4-(4-Fluor-benzamido)-piperidino]-propylchlorid | aus | 46 | 203—204 (Isopropanol) |
| c) | 9-(3-[4-(2-Methoxy-benzamido)-piperidino]-propyl)-adenin aus Adenin und 3-[4-(2-Methoxy-benzamido)-piperidino]-propylchlorid | aus | 66 | 139—141 (Ether) |
| d) | 9-(3-[4-(4-n-Butoxy-benzamido)-piperidino]-propyl)-adenin aus Adenin und 3-[4-(4-n-Butoxy-benzamido)-piperidino]-propylchlorid | aus | 32 | 208—210 (Isopropanol) |
| e) | 9-(3-[4-(2-Methyl-benzamido)-piperidino]-propyl)-adenin aus Adenin und 3-[4-(2-Methyl-benzamido)-piperidino]-propylchlorid | aus | 35 | 182—183 (Isopropanol) |

| | Bezeichnung | | Ausbeute % | Schmp. °C (Lösungsmittel) |
|---|---|---|---|---|
| f) | 9-(3-[4-(4-Methyl-benzamido)-piperidino]-propyl)-adenin<br>Adenin und 3-[4-(4-Methyl-benz-amido)-piperidino]-propylchlorid | aus | 25 | 220—222 (Isopropanol) |
| g) | 9-(3-[4-(3-Trifluoromethyl-benz-amido)-piperidino]-propyl)-adenin<br>Adenin und 3-[4-(3-Trifluorme-thyl-benzamido)-piperidino]-propylchlorid | aus | 39 | 176—177 (Essigester) |
| h) | 9-(3-[4-(4-t-Butyl-benzamido)-piperidino]-propyl)-adenin<br>Adenin und 3-[4-(4-t-Butyl-benzamido)-piperidino]-propyl-chlorid | aus | 49 | 186—187 (Ether) |
| i) | $N^6$-Methyl-9-[3-(4-Benzamido-piperidino)-propyl]-adenin-<br>$N^6$-Methyladenin und 3-(4-Benz-amido-piperidino)-propylchlorid | aus | 31 | 212—214 (Isopropanol/Ether) |
| j) | $N^6$-(2-Hydroxyethyl)-9-[3-(4-Benz-amido-piperidino)-propyl]-adenin<br>$N^6$-(2-Hydroxyethyl)-adenin und 3-(4-Benzamido-piperidino)-propyl-chlorid | aus | 46 | 154—155 (Isopropanol) |
| k) | $N^6$-n-Butyl-9-[3-(4-Benzamido-piperidino)-propyl]-adenin<br>$N^6$-n-Butyladenin und 3-(4-Benz-amido-piperidino)-propylchlorid | aus | 46 | 190—192 (Isopropanol/Ether) |
| l) | 6-Dimethylamino-9-[3-(4-Benz-amido-piperidino)-propyl]-purin<br>6-Dimethylamino-purin und 3-(4-Benzamido-piperidino)-propylchlorid | aus | 61 | 105—107 Essigester/Ether |
| m) | 6-Piperidino-9-[3-(4-Benzamido-piperidino)-propyl]-purin<br>6-Piperidino-purin und 3-(4-Benzamido-piperidino)-propyl-chlorid | aus | 69 | 144—145 (Isopropanol) |
| n) | 9-[3-(4-Acetamido-piperidino)-propyl]-adenin<br>Adenin und 3-(4-Acetamido-piperidino)-propylchlorid | aus | 34 | 213—215 (Isopropanol/Ether) |

Beispiel 3

*9-[3-(4-Cinnamoylamido-piperidino)-propyl]-adenin*

Zu einer Mischung aus 6.2 g (0.02 mol) 9 - [3 - (4 - Amino-piperidino) - propyl] - adenin und 100 ml 1 N Natronlauge tropft man 3.7 g (0.022 mol) Zimtsäurechlorid, rührt 5 Stunden bei Raumtemperatur, filtriert und kristallisiert aus Isopropanol um. Man erhält 3.2 g 9 - [3 - (4 - Cinnamoylamido - piperidino) - propyl] - adenin (39% d. Th.) vom Schmp. 217—219°C.

Das als Reaktionskomponente verwendete 9 - [3 - (4 - Amino - piperidino) - propyl] - adenin kann wie folgt erhalten werden:

Eine Mischung aus 32.0 g (0.085 mol) 9 - [3 - (4 - Benzamido - piperidino) - propyl] - adenin und 500 ml 5 N Salzsäure wird 6 Stunden zum Rückfluss erhitzt. Man lässt abkühlen, wäscht mit Ether, stellt alkalisch, extrahiert mit Methylenchlorid und engt den Extrakt ein. Nach Umkristallisation des Rückstands aus Isopropanol erhält man 14.6 g 9 - [3 - (4 - Amino - piperidino) - propyl] - adenin (62% d. Th.) vom Schmp. 140—142°C (Hydrochlorid 318—320°C).

Beispiel 4

In analoger Weise wie in Beispiel 3 beschrieben erhält man:

| | Bezeichnung | | Ausbeute % | Schmp. °C (Lösungsmittel) |
|---|---|---|---|---|
| a) | 9-(3-[4-(4-Chlor-benzamido)-piperidino]-propyl)-adenin | aus | 41 | 203—205 (Isopropanol/Ether) |
| | 9-[3-(4-Amino-piperidino)-propyl]-adenin und 4-Chlor-benzoylchlorid | | | |
| b) | 9-(3-[4-(2-Hydroxy-benzamido)-piperidino]-propyl)-adenin | aus | 19 | 193—195 (Aceton) |
| | 9-[3-(4-Amino-piperidino)-propyl]-adenin und Salicylsäurechlorid | | | |
| c) | 9-(3-[4-(4-Nitro-benzamido)-piperidino]-propyl)-adenin | aus | 28 | 142—143 (Isopropanol) |
| | 9-[3-(4-Amino-piperidino)-propyl]-adenin und 4-Nitro-benzoylchlorid | | | |
| d) | 9-(3-[4-(Phenyl-acetamido)-piperidino]-propyl)-adenin | aus | 32 | 190—192 (Essigester) |
| | 9-[3-(4-Amino-piperidino)-propyl]-adenin und Phenylessigsäurechlorid | | | |
| e) | 9-(3-[4-(2-Phenyl-propionamido)-piperidino]-propyl)-adenin | aus | 27 | 185—187 (Essigester) |
| | 9-[3-(4-Amino-piperidino)-propyl]-adenin und 2-Phenyl-propionsäurechlorid | | | |
| f) | 9-(3-[4-(Furan-2-carbonylamido)-piperidino]-propyl)-adenin | aus | 39 | 201—202 (Isopropanol) |
| | 9-[3-(4-Amino-piperidino)-propyl]-adenin und Furan-2-carbonsäurechlorid | | | |

| | Bezeichnung | | Ausbeute % | Schmp. °C (Lösungsmittel) |
|---|---|---|---|---|
| g) | 9-(3-[4-Thiopen-2-carbonylamido)-piperidino]-propyl)-adenin aus 9-[3-(4-Amino-piperidino)-propyl]-adenin und Thiopen-2-carbonsäurechlorid | aus | 55 | 210—212 (Isopropanol) |
| h) | 9-(3-[4-(Pyridin-3-carbonylamido)-piperidino]-propyl)-adenin aus 9-[3-(4-Amino-piperidino)-propyl]-adenin und 3-(Isobutoxycarbonyloxy--carbonyl)pyridin | aus | 29 | 203—205 (Isopropanol/Ether) |
| i) | 9-(3-[4-(4-Aminocarbonyl-benzamido)-piperidino]-propyl)-adenin aus 9[3-(4-Amino-piperidino)-propyl]-adenin und 4-(Isobutoxycarbonyloxycarbonyl)-benzamid | aus | 39 | 278—280 (Aceton) |
| j) | 9-[3-(4-Benzolsulfonamido-piperidino)-propyl]-adenin aus 9-[3-(4-Amino-piperidino)-propyl]-adenin und Benzolsulfochlorid | aus | 23 | 175—177 (Essigester) |
| k) | 9-[3-(4-Pivaloylamido-piperidino)-propyl]-adenin aus 9-[3-(4-Amino-piperidino)-propyl]-adenin und Pivalinsäurechlorid | aus | 53 | 190—192 (Isopropanol/Ligroin) |
| l) | 9-[3-(4-Cyclopropancarbonylamido-piperidino)-propyl]-adenin aus 9-[3-(4-Amino-piperidino)-propyl]-adenin und Cyclopropancarbonsäurechlorid | aus | 27 | 202—204 (Essigester) |
| m) | 9-[3-(4-Cyclohexancarbonylamido-piperidino)-propyl]-adenin aus 9-[3-(4-Amino-piperidino)-propyl]-adenin und Cyclohexancarbonsäurechlorid | aus | 36 | 231—233 (Isopropanol/Ligroin) |
| n) | 9-(3-[4-(3-Phenyl-propionamido)-piperidino]-propyl)-adenin aus 9-[3-(4-Amino-piperidino)-propyl]-adenin und 3-Phenyl-propionsäurechlorid | aus | 44 | 182—183 (Essigester) |

| Bezeichnung | | Ausbeute % | Schmp. °C (Lösungsmittel) |
|---|---|---|---|
| o) 9-(3-[4-(3,4-Dimethoxy-cinnamoylamido)-piperidino]-propyl)-adenin aus 9-[3-(4-Amino-piperidino)-propyl]-adenin und 3,4-Dimethoxy-zimtsäurechlorid | aus | 29 | 218—219 (Isopropanol) |

Beispiel 5

*9-(3-[4-(4-Amino-benzamido)-piperidino]-propyl)-adenin*

Eine Mischung aus 3.0 g (0.007 mol) 9 - (3 - [4 - (4 - Nitro - benzamido) - piperidino] - propyl) - adenin, 50 ml Methanol, 25 ml Tetrahydrofuran und 1 g Raney-Nickel hydriert man 2 Stunden bei Raumtemperatur und 1 bar Wasserstoffdruck. Danach wird filtriert, eingeengt und der Rückstand mit Essigester verieben.

Man erhält 2.4 g 9 - (3 - [4 - (4 - Amino - benzamido) - piperidino] - propyl) - adenin (86% d. Th.) vom Schmp. 247—248°C.

Beispiel 6

*$N^6$-n-Butyl-9-[3-(4-Benzamido-piperidino)-propyl]-adenin*

Zu einer Lösung von 12.0 g (0.03 mol) 6 - Chlor - 9 - [3 - (4 - benzamido - piperidino) - propyl] - purin in 100 ml n-Propanol fügt man 50 ml n-Butyl-amin und erhitzt 6 Stunden zum Rückfluss. Darauf wird im Vakuum eingeengt und der Rückstand mit Essigester extrahiert. Nach Abdampfen des Lösungsmittels und Umkristallisation aus Isopropanol/Ether erhält man 6.8 g $N^6$ - n - Butyl - 9 - [3 - (4 - Benzamido - piperidino) - propyl]-adenin (52% d. Th.) vom Schmp. 190—192°C.

Beispiel 7

*9-(3-[4-(4-t-Butyl-benzamido)-piperidino]-propyl)-adenin - hydrochlorid*

Eine Suspension von 1.0 g 9 - (3 - [4 - (4 - t - Butyl - benzamido) - piperidino] - propyl) - adenin in 10 ml Ethanol versetzt man mit überschüssiger etherischer Chlorwasserstofflösung. Beim Versetzten der Lösung mit Ether erhält man 0.95 g 9 - (3 - [4 - (4 - t - Butyl - benzamido) - piperidino] - propyl) - adenin - hydrochlorid (87 % d. Th.) vom Schmp. 285—287°C.

**Patentansprüche**

1. Purin-Derivate der allgemeinen Formel I

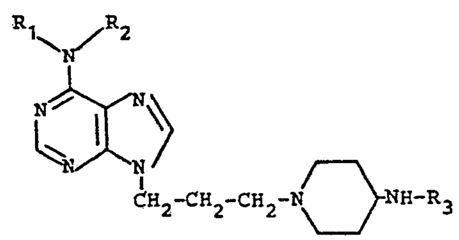

(I)

in welcher

$R_1$ Wasserstoff oder eine Alkylgruppe mit 1 bis 6 Kohlenstoffatomen,

$R_2$ Wasserstoff, eine Aryl- oder eine Alkylgruppe mit 1 bis 6 Kohlenstoffatomen, die gegebenenfalls durch Hydroxyl substituiert sein kann, wobei $R_1$ und $R_2$ auch zusammen mit dem Stickstoffatomen einen heterocyclischen Ring bilden können, und

$R_3$ eine Acylgruppe oder Wasserstoff bedeuten, sowie deren Salze mit pharmakologisch verträglichen Säuren.

2. Verfahren zur Herstellung von Purin-Derivaten der allgemeinen Formel I gemäß Anspruch 1, wobei $R_1$, $R_2$ und $R_3$ die hierin angegebene Bedeutung haben, dadurch gekennzeichnet, daß man in an sich bekannter Weise eine Verbindung der allgemeinen Formel II

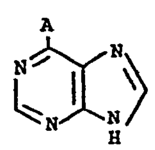

(II)

in der A einen reaktiven Rest oder die

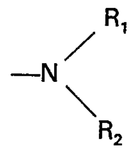

-Gruppe bedeutet,
mit einer Verbindung der allgemeinen Formel III

$$X—CH_2—CH_2—CH_2—Y \qquad (III),$$

in der
X und Y reaktive Reste bedeuten und einer Verbindung der allgemeinen Formel IV

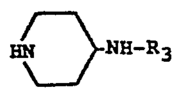

(IV)

umsetzt,
wobei für den Fall, dass A eine reaktive Gruppe bedeutet, die

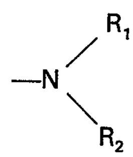

-Gruppe nachträglich eingeführt werden muss, anschliessend gewünschtenfalls die Gruppe $R_3$ durch bekannte Verfahren in eine andere Gruppe $R_3$ umwandelt,
sowie das erhaltene Reaktionsprodukt gewünschtenfalls in ein pharmakologisch verträgliches Salz überführt.

3. Verwendung von Substanzen der allgemeinen Formel I und deren Salzen mit pharmakologisch verträglichen Säuren zur Herstellung von Arzneimitteln mit antiallergischer, entzündungswidriger und antiödematöser Wirkung.

4. Arzneimittel, gekennzeichnet durch einen Gehalt an Verbindungen der allgemeinen Formel I und deren Salze mit pharmakologisch verträglichen Säuren.

**Revendications**

1. Dérivés de purine de formule générale I

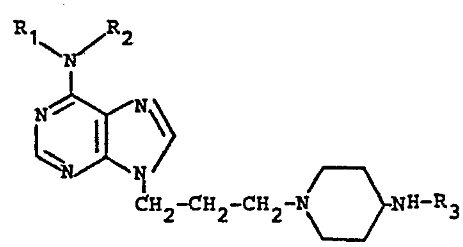

(I)

dans laquelle:
$R_1$ est un atome d'hydrogène ou un groupe alkyle ayant de 1 à 6 atomes de carbone.
$R_2$ es de l'hydrogène, un groupe aryle ou alkyle ayant de 1 à 6 atomes de carbone, éventuellement substitué par une fonction hydroxyle, $R_1$ et $R_2$ pouvant aussi former ensemble avec les atomes d'azote un noyau hétérocyclique, et
$R_3$ est un groupe 'acyle ou de l'hydrogène, ainsi que leurs sels d'acides pharmacologiquement tolérables.

2. Procédé pour la préparation de dérivés de purine de formule générale I selon la revendication 1, $R_1$, $R_2$ et $R_3$ ayant la signification ci-dessus indiquée, caractérisé en ce que l'on fait réagir de façon en soi connue, un composé de formule générale II,

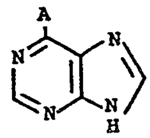

(II)

dans laquelle:
A est un reste réactif ou le groupe

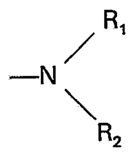

avec un composé de formule générale III

$$X—CH_2—CH_2—CH_2—Y \qquad (III)$$

dans laquelle:
X et Y sont des restes réactifs, et avec un composé de formule générale IV

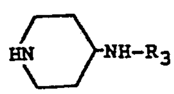

le groupe

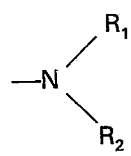

devant être introduit ultérieurement dans le cas où A est un groupe réactif,
et en ce que l'on transforme consécutivement, si l'on désire, le group $R_3$ selon un procédé connu en un autre groupe $R_3$,
et en ce que l'on transforme le produit de réaction obtenu éventuellement en un sel pharmacologiquement tolérable.

3. Utilisation des substances de formule générale I et de leurs sels d'acides pharmacologiquement tolérables pour la fabrication de médicaments ayant une activité anti-allergique, anti-infectieuse et anti-oedémateuse.

4. Médicaments caractérisé en ce qu'ils ont une teneur en composés de formule générale I ou de leurs sels d'acides pharmacologiquement tolérables.

**Claims**

1. Purine derivatives of the general formula I

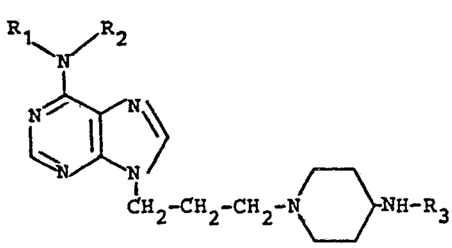

(I).

in which $R_1$ signifies hydrogen or an alkyl group with 1 to 6 carbon atoms, $R_2$ hydrogen, an aryl or an alkyl group with 1 to 6 carbon atoms, which can optionally be substituted by hydroxyl, whereby $R_1$ and $R_2$, together with the nitrogen atom, can together also form a heterocyclic ring, and $R_3$ an acyl group or hydrogen, as well as their salts with pharmacologically acceptable acids.

2. Process for the preparation of purine derivatives of the general formula I according to claim 1, whereby $R_1$, $R_2$ and $R_3$ have the meaning given herein, characterised in that, in per se known manner, one reacts a compound of the general formula II

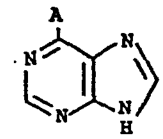

in which A signifies a reactive residue or the

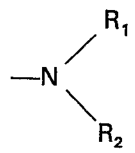

group, with a compound of the general formula III

$$X—CH_2—CH_2—CH_2—Y \qquad (III),$$

in which X and Y signify reactive residues, and a compound of the general formula IV

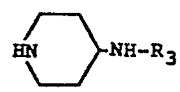

(IV)

whereby, for the case in which A signifies a reactive group, the

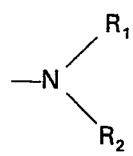

group must be subsequently introduced, subsequently, if desired, one converts the $R_3$ group into another group $R_3$ by known processes, as well as, if desired, converts the reaction product obtained into a pharmacologically acceptable salt.

3. Use of substances of the general formula I and of their salts with pharmacologically acceptable acids for the preparation of medicaments with anti-allergic, anti-inflammatory and anti-edematous action.

4. Medicaments, characterised by a content of compounds of the general formula I and of their salts with pharmacologically acceptable acids.